(19) Europäisches Patentamt
European Patent Office
Office européen des brevets

(11) Publication number: 0 230 084 A1

(12) **EUROPEAN PATENT APPLICATION**

(21) Application number: 86202377.7

(22) Date of filing: 23.12.86

(51) Int. Cl.[4]: G 01 L 9/12

(30) Priority: 24.12.85 NL 8503574

(43) Date of publication of application: 29.07.87 Bulletin 87/31

(84) Designated Contracting States: DE FR GB IT NL

(71) Applicant: Sentron v.o.f., Oosteinde 8, NL-9301 LJ Roden (NL)

(72) Inventor: Ligtenberg, Henrikus Cornelis Geert, Johan Vermeerstraat 15, NL-9312 PZ Nietap (NL)

(74) Representative: Smulders, Theodorus A.H.J. et al, Vereenigde Octrooibureaux Nieuwe Parklaan 107, NL-2587 BP 's-Gravenhage (NL)

(54) **Blister FET pressure sensor.**

(57) A capacitive pressure sensor is presented employing a field effect transistor including a semiconductor substrate (10) having a source region (7) and a drain region (6) with a channel region therebetween. A blister structure (9) is mounted on the substrate and which has walls defining a hermetically sealed cavity (8), exhibiting a low pressure. The cavity overlies the channel region and at least portions of the source and drain regions. The cavity has a flexible roof (14) containing conductive material serving as a flexible capacitor plate which is spaced from and overlies the channel region. Displacement of the flexible roof, due to applied pressure, relative to the channel region varies the transconductance thereof.

P
8
14
9
3
12
D
S
10
5

EP 0 230 084 A1

Title: Blister FET pressure sensor

This invention relates to the art of pressure sensors employing FET components and, more particularly, to an improved pressure sensor employing the capacitive effects of a movable capacitor plate in which the FET component is incorporated directly into the capacitive component.

The article 'Micro-Miniature Solid-State Capacitive Blood Pressure Transducer With Improved Sensitivity' by Wolf D.Frobenius et al in IEEE Transactions on Biomedical Engineering, July 1973, pages 312-314, states that the operation of catheter tip pressure sensors is often based on the piezoresistive effect of a pressure-responsive mechanism. The article further establishes that the capacitive effect of a moving capacitor plate has not yet been used as a basis for the operation of catheter tip pressure sensors, mainly as it has appeared difficult to realize the sensor geometry required for such use and as the high output impedance of a small-size capacitor impedes detection.

On the other hand, the Frobenius et al. article mentions a high sensitivity, a lesser power dissipation as well as a lesser sensitivity to changes in temperature as advantages of a capacitive pressure sensor over a pressure sensor operating on the basis of the piezoresistive effect.

To permit the use of a capacitive effect as a basis for the operation of a pressure sensor to be mounted in the tip of a catheter, Frobenius et al. proposes that such a pressure sensor be based on the operation of an air capacitor comprising a flexible membrane etched in a silicon substrate, in which a golden ring is disposed circumferentially of the membrane and a metallic plate is mounted in spaced, parallel relation to the membrane. A FET structure is provided at the center of the membrane and the metal plate is provided with a stem through which this plate rests directly on the FET gate.

In addition to the Frobenius article, the relevant prior art includes French patent no. 1,568,487 which corresponds with US patent to Eichelberger, 3,440,873; French patent no. 1,461,244 which corresponds with British patent no. 1,088,793; the published European patent application 0-087-264; and, the US patent to Shimada et al., 4,495,820. Of the noted references, the patent to Eichelberger appears particularly pertinent, since it discloses a field effect transistor including a semiconductor substrate having source and drain regions and a channel region between the source and drain regions. A movable gate plane is spaced from and overlies the conduction channel and is free to move with respect thereto so that the conductivity of the channel is a function of any deflection of the gate plane with respect to the channel region. However, the gate plane is supported in spaced relationship to the substrate by means of spacer insulators in a laminated construction, which, in practice, is difficult to manufacture and which does not necessarily obtain a hermetically sealed structure so that the space between the substrate and the movable gate plane defines a sealed cavity. Moreover, there is no teaching in Eichelberger of insuring that the cavity is constructed to avoid internal ionic contamination to prevent leakage current flow and hence an inherent instability.

British patent No. 1,088,793 is similar to Eichelberger, supra, in that it discloses a movable gate electrode located on a diaphragm spaced from the channel region of a FET structure embedded in the substrate. As in Eichelberger, there is no teaching of a hermetically sealed cavity constructed so as to avoid ionic contamination.

The European patent application no. 0-087-264 (which corresponds with US patent no. 4,480,488) is directed to a pressure sensor employing a field effect transistor structure having a layer of piezoresistive material between the channel region and the gate electrode and, hence, does not disclose or suggest the use of a movable capacitor plate. The US patent 4,495,820 discloses a capacitive pressure sensor similar to that in Eichelberger, supra, wherein a movable electrode is spaced from a substrate containing an amplifier. However, as in Eichelberger, there is no teaching of a cavity between the substrate and the movable electrode and which is hermeticallly sealed in such a fashion as to avoid ionic contamination.

Accordingly, it is an object of the present invention to provide a structurally novel capacitive pressure sensor adapted for manufacture by techniques that are fully compatible with present day FET technology.

It is a still further object of the present invention to provide a movable capacitor plate FET pressure sensor wherein the space between the capacitor plate and the substrate is a hermetically sealed cavity defined by a flexible blister wall enclosing the cavity having essentially zero pressure and being constructed in such a manner as to avoid ionic contamination within the cavity to thereby prevent leakage current.

To achieve this object in accordance with the invention, a capacitive pressure sensor is employed which includes a field effect transistor including a semiconductor substrate having a source region and a drain region and a channel region between said source and drain regions. A blister structure is mounted on the substrate and it has walls defining a hermetically sealed cavity exhibiting a low pressure, less than atmospheric pressure, and which overlies at least portion of the source and drain regions as well as the channel region therebetween. The cavity walls include a flexible roof containing a layer of conductive material serving as a movable capacitor plate which is spaced from and overlies the channel region so as to vary the transconductance thereof as a function of any displacement of the flexible roof relative to the channel region as caused by pressure applied to the roof.

The foregoing and other objects and advantages of the invention will become more apparent from the following description of the preferred embodiment of the invention as taken in conjunction with the accompanying drawings, wherein:

Figs. 1A through 1E schematically show a number of stages in the manufacture of a hermetically sealed blister-like structure for a pressure sensor according to the invention;

Figs. 2A and 2B show two circuit configurations employing the pressure sensor in accordance with the invention;

Fig. 3 is a graphical illustration showing the variation in transconductance of the FET structure with variations in applied pressure;

Fig. 4 is a top or plan view of a second embodiment of the pressure sensor in accordance with the present invention;

Fig. 5 is a sectional view taken along line 5-5 looking in the direction of the arrows of Fig. 4;

Fig. 6 is a sectional view taken along line 6-6 looking in the direction of the arrows in Fig 4;

Fig. 7 is a sectional view taken along line 7-7 looking in the direction of the arrows of Fig. 4;

Fig. 8 is a schematic illustration showing one application of the invention in the form of a catheter tip transducer mounted on the distal end of a catheter for use in monitoring blood pressure or the like in a living body; and

Fig. 9 is an enlarged, partly in section, view illustrating the catheter tip transducer.

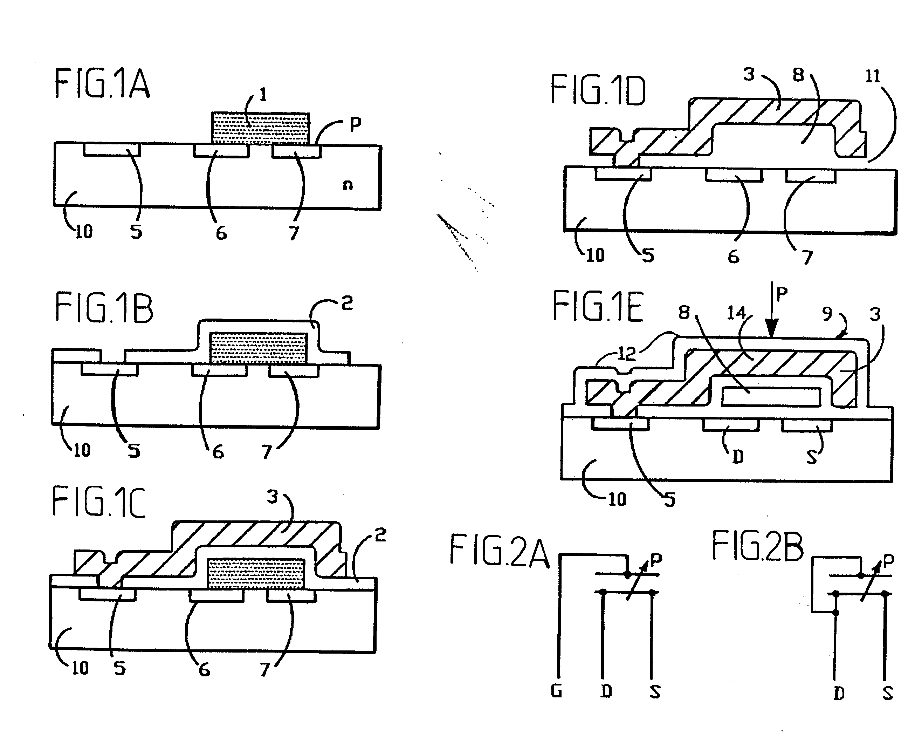

Reference is now made to Figs. 1A-1E which show in stages the steps in manufacturing the blister FET pressure sensor in accordance with the present invention. In Fig. 1A, there is provided a semiconductive substrate 10 of n-type silicon having regions 6 and 7 of P-type silicon diffused therein. Regions 6 and 7 serve, respectively, as the drain and source regions. After definition of these drain and source regions, a spacer 1 of silicon dioxide is deposited over the gate area (the area or channel region between the drain and source regions). Spaced laterally to the left, as viewed in Fig. 1A, there is provided a P-type silicon region 5 diffused in the substrate. The silicon dioxide block or spacer 1 is etched away in a later stage in the manufacture of the sensor.

Referring now to Fig. 1B, there is shown the second stage of manufacture. Here, a second spacer layer 2 is applied over the top surface of substrate 10, as well as over the spacer 1, but leaving the region 5 exposed. The second spacer layer 2 will also be etched away in a later stage of manufacture.

The third stage in the manufacture of the pressure sensor is illustrated in Fig. 1C wherein a polysilicon layer 3 is applied over spacer layer 2. This polysilicon layer 3 makes electrical contact with the exposed region 5.

In the next stage, as shown in Fig. 1D, the silicon dioxide spacers 1 and 2 are etched away laterally by way of tunnel 11 defined by the spacer layer 2. The spacers may be etched away completely by means of hydrofluoric acid to leave a cavity 8 communicating with the ambient by means of tunnel 11. The geometry of the tunnel 11, for example, may be on the order of 200 A high and 50 micrometers wide. By subsequently subjecting the entire structure to oxidation in pure oxygen (Fig. 1E), the entire blister-like structure 9 is coated with a silicon dioxide layer 12 which seals off the tunnel 11 (Fig. 1E). This oxidation results in cavity 8 being internally and externally coated with a layer of silicon dioxide, causing the oxygen in the cavity to be consumed to a very low pressure, on the order of 0 to a negative pressure. This oxidation takes place at a temperature on the order of 1000 to 1150°C resulting in a hermetically sealed cavity. Moreover, annealing at this high temperature prevents any water to remain in the cavity, it being evaporated or subjected to a reduction process. Without water in the cavity, ionic contamination is avoided, thereby minimizing the prospect of any leakage currents.

Regions 5, 6 and 7 can be subsequently connected, as shown in Fig. 2A or 2B, wherein the regions 6 and 7 respectively serve as the drain and source of the FET structure, and region 5 serves as the gate electrode. The circuit configuration of Fig. 2A permits a direct measurement of the transconductance $G_m$. The circuit configuration of Fig. 2B results in a reduction of the number of external contacts, but also in a device operating as a pressure-responsive resistor.

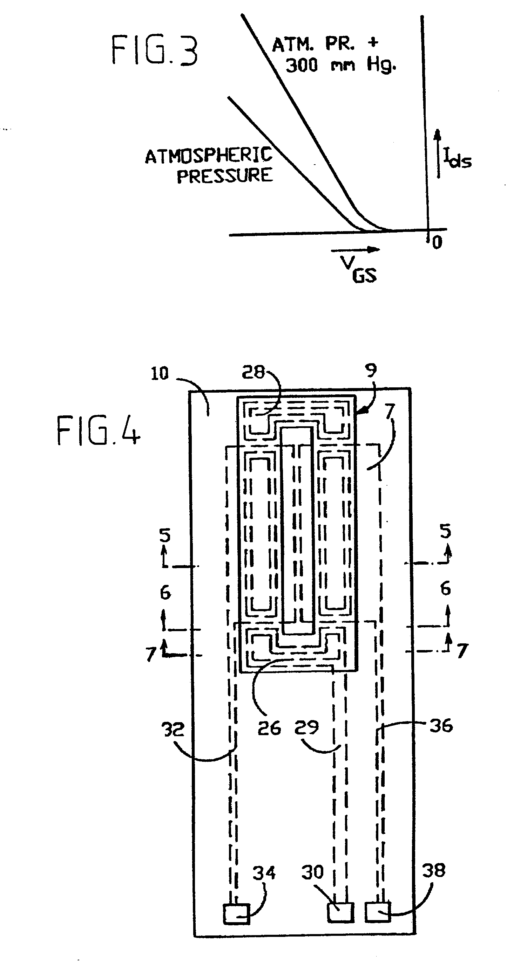

As is seen from Figs. 1E, 2A and 2B, the blister FET structure 9 responds to pressure, such as that indicated by the arrow P acting on the polysilicon blister roof 14 to cause the roof to be deflected toward the substrate in an area directly over the channel region between the drain and source regions. This causes an increase in the capacitance. This, in turn, changes the transconductance of the FET, which increases linearly with capacitance per unit area at a point midway between the drain and source. This is illustrated more graphically with respect to Fig. 3, which shows the blister sensor characteristics with the variations in transconductance with an increase of pressure from that of atmospheric pressure.

In view of the foregoing, it is seen, in accordance with the present invention, that a pressure sensor has been provided which is characterized in that the capacitive component is a blister structure having a flexible blister wall enclosing a space or cavity of greatly reduced pressure. The blister structure is mounted on a rigid silicon chip or substrate 10 which is provided with a drain region 6 and a source region 7 pertaining to the FET structure within the area enclosed by the blister wall. The blister-like structure to be used in accordance with the invention may be mounted on a silicon chip by employing a known per se method described by W. Guckel and D.W. Burns in 'Workshop on Micromachining and Micro-packaging of Transducers', November 7-9, 1984, Case Western University of Cleveland, Ohio. By employing this method, the blister-like structure is achieved by etching away a block shaped spacer, i.e., the spacer 1 described hereinbefore. This spacer is disposed on the silicon substrate and fully surrounded by a layer of material that will consitute the blister wall once the spacer has been etched away laterally, after which etching the resultant blister-like structure is subjected to a treatment for hermetically sealing the same.

This technique can be applied to various combinations of materials, for example, combinations of polysilicon as the material for the blister wall to be formed and silicon dioxide for the spacer to be wasted. An aluminum alloy may be employed for the blister wall and nickel may be employed for the spacer. Alternatively, silicon dioxide may be used for the blister wall and metal, such as aluminum, may be employed for the spacer.

With a negative pressure in the cavity 8, even as low as 0 pressure, the walls of the blister structure 9 will not collapse because of the outwardly bent shape of the wall (cylindrical or, in cross section, dome-like shape) and particularly because of compressive built-in stresses which makes the wall behave similar to that of prestressed concrete. The origin of the internal stresses is, for example, mismatches in thermal-expansion coefficients or 'intrinsic stress' caused by nucleation and grain growth.

An alternative process for sealing the blister structure 9 in Fig. 1E may be accomplished by annealing the blister structure in oxygen at a temperture in the range of 1000° to 1150°C until the blister structure is approximately sealed. Thereafter, a definite sealing is realised by 'low pressure chemical vapor deposition' techniques, with the deposition of a $Si_3N_4$ layer, while keeping the approximately sealed blister structure in a reaction mixture containing dichloro silane and ammonia at a temperature of about 600° to 650°C and low pressure. Also, with this alternative sealing process, the pressure in the cavity may be guaranteed to be lower than 0,1 torr.

In the embodiment described in Fig. 1E, the blister wall is the polysilicon layer 3 and the spacer (1') is a silicon dioxide block. As discussed earlier herein, the blister wall may, instead, be made from silicon dioxide and the spacer may be made from alumina. In such case, then, the sealing step after having etched away the aluminum spacer, may be carried out by means of a chemical vapor deposition technique of silicon dioxide ($SiO_2$) in which case the cavity, when sealed, will not have a negative pressure, or by means of the earlier mentioned low pressure 'vapor deposition' technique, depositing a $Si_3N_4$ layer, and finally leaving the cavity with a pressure lower than 0,1 torr.

In the medical field, it has been known to employ catheter tip pressure sensors wherein a piezoresistive-type transducer is mounted at the end of a catheter. Typically, such pressure transducers require that an air reference lumen be employed in the catheter so that a pressure reading is made with respect to atmospheric pressure. By employing a blister structure FET pressure sensor as described herein, the sensor will yield an absolute pressure reading. For a miniature catheter tip pressure sensor, this is an attractive approach, as it will also eliminate the air reference lumen normally employed in catheter pressure tip sensor. Such a structure, then, permits realization of pressure sensors of extremely small diameter. Heretofore, a catheter tip pressure sensor had been limited in size to catheter diameters of greater than one millimeter (nm). This, in large messure, is because of the requirement of a catheter having an air lumen. By employing a blister FET pressure sensor as discussed herein which does not require en air reference lumen, as absolute pressure is being measured, a catheter tip pressure sensor may now be realized for use with catheter diameters below 1 mm. An example of such a catheter is presented hereinafter.

Reference is now made to Figs. 4, 5, 6 and 7 which illustrate another embodiment of the blister FET structure in greater detail than that presented heretofore with respect to the structure shown in Fig. 1E. To facilitate an understanding of this description, like components in Fig. 1E and in Figs. 4-7 will be identified with like character references.

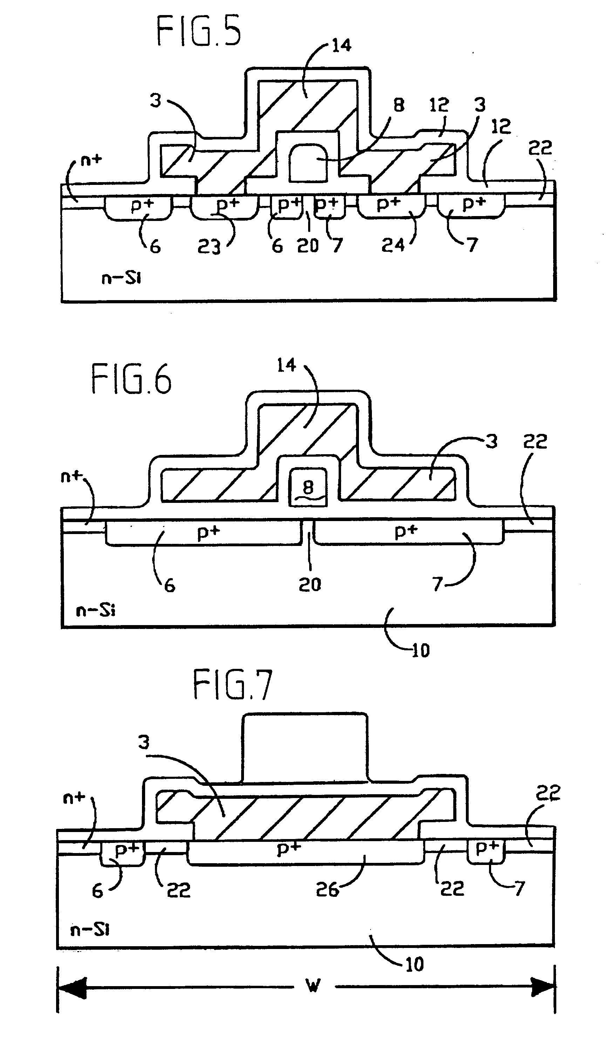

A shown in Figs. 4-7, the polyblister FET structure 9 is securely mounted on a silicon substrate 10. The manner of construction takes the same fashion as that discussed hereinbefore with respect to Figs. 1A-1E. It is to be noted that the width W (as best seen in Fig. 7) of the substrate block is substantially less than 1 mm and, for example, may be on the order of 600 microns. This permits the substrate block, together with the blister FET pressure sensor structure, to be fitted within a catheter having an inner diameter of less than 1 mm.

As is best seen in Figs. 4 and 6, the drain region 6 and the source region 7 take the form of elongated, somewhat rectangular P+silicon diffusions located in the upper surface of the substrate 10. These P+ regions 6 and 7 extend parallel to each other and are transversely spaced apart, leaving therebetween a gate area or channel region 20. This channel region 20 is located below the cavity 8 and is aligned with the middle of the roof portion 14 of the polysilicon layer 3. In this embodiment, there is also illustrated a channel stopper layer 22, which is located on the upper surface of the substrate 10. This channel stopper layer may take the form of an N+ diffusion which extends across the entire upper surface of the substrate, except for the areas at which the P+ diffusions are located and except for the area between the source and drain regions. This is done to restrict the channel region 20 to the area between the source and drain regions 6 and 7.

As is best seen in Figs. 5 and 7, the drain region 6 and the source region 7 each have a rectangular opening into which an elongated rectangular shaped blister wall support is provided. Thus, in the drain region 6 there is provided a blister wall support 22 and in the source region 7 there is provided a blister wall support 24. The supports 23 and 24 may take the form of P+ diffusions in the upper surface of the substrate 10. Each of these supports is electrically isolated from the drain and source regions by virtue of the (reversely biased) p-n diode that is formed with the substrate 10 and the channel stopper 22. These supports 23 and 24 provide electrical connection and mechanical support for the polysilicon blister wall or layer 3 while electrically isolating layer 3 from the drain and source regions.

The blister wall supports 23 and 24 may be considered as side wall supports. In addition to the side wall supports, there is also provided a pair of end wall supports for providing mechanical support for the end walls of the blister structure, while also electrically isolating the polysilicon layer 3 from the drain and source regions. These end wall supports are illustrated in Fig. 4 as a U-shaped end wall support 26 and an inverted U-shaped en wall support 28. Each accomplishes the same function and Fig. 7 is a cross sectional view showing end wall support 26 in greater detail. Although only the details of the end wall support 26 are discussed hereinbelow, it is to be understood that end wall support 28 is constructed in the same manner.

As is shown in Fig. 7, the end wall support 26 takes the form of a P+ diffusion in the upper surface of the substrate 10. The support 26 is electrically isolated from the drain region 6 and the source region 7 by means of the (reversely biased) p-n diode that is formed with the substrate 10 and the channel stopper 22. The end wall support 26 is mechanically and electrically connected with the polysilicon layer 3 and, hence, is in electrical communication with the roof area 14 of the blister structure. The roof area 14 serves as movable capacitor plate or gate plane in this structure. Consequently, a conductive strip 29 may be extended from support 26, in the manner shown in Fig. 4, to a gate contact pad 30 providing external connections to the FET structure. This conductive strip 29 may take the form of an elongate P+ diffusion extending from support 26 to the contact pad 30. In a similar manner, a conductive strip 32 may be extended from the drain region 6 to a drain contact pad 34. Also, in a similar fashion, another conductive strip 36 extends from the source region 7 to a source contact pad 38.

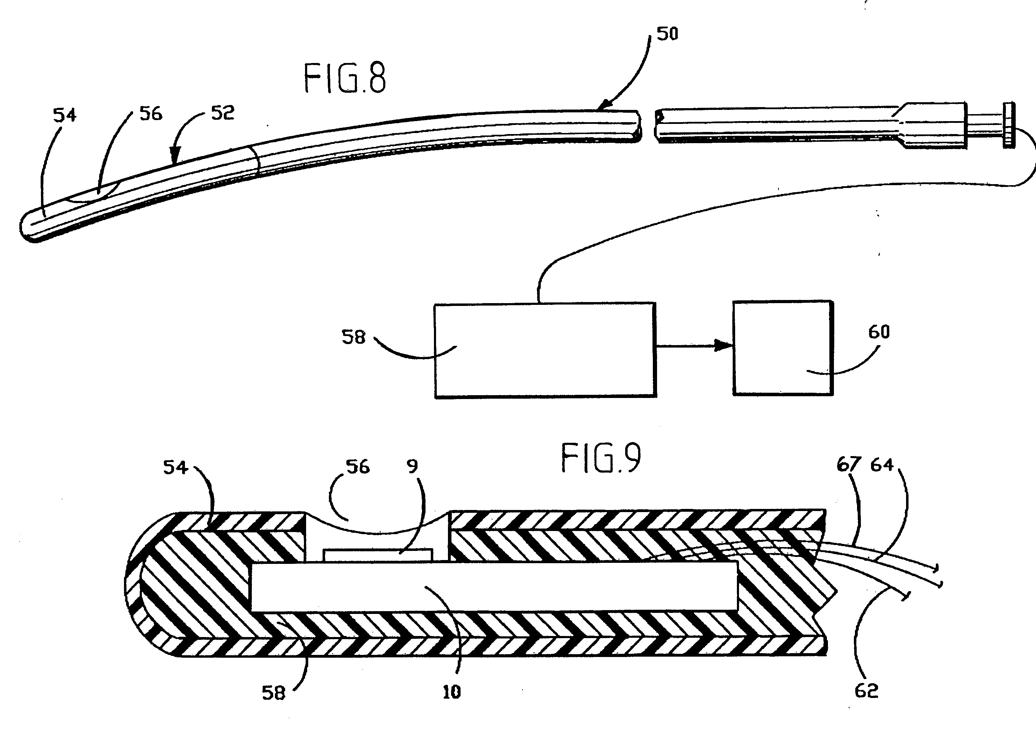

Having now described the embodiment shown in Figs. 4-7, attention is directed to an application of the invention as applied to measurement of blood pressure within a blood vessel of a living body. Reference is now made to Fig. 8, which illustrate an elongated, flexible, single lumen catheter 50 having a catheter tip pressure transducer 52, employing the present invention, adhered to the catheter's distal end. The catheter tip pressure transducer 52 includes a relatively rigid, tubular housing 54 having a side port pressure inlet aperture 36 therein for communicating blood pressure at the site of interest to the blister FET pressure sensor located within the housing 54. The blister FET pressure sensor has a flexible roof 14 which deforms in dependence upon the pressure being monitored. This deformation causes an increase in capacitance and thereby causes a corresponding change in the transductance of the FET structure. This change in transconductance is measured by interconnecting the gate contact pad 30 as well as the drain contact pad 34 and the source contact pad 38 with suitable electrical conductors which extend through the lumen of the catheter 50 beyond the proximal end thereof to a suitable power supply and detector circuit 58. The resulting detection may then be recorded or otherwise read out, as with a suitable meter 60.

In operation, the catheter with the catheter tip pressure transducer is inserted within a blood carrying vessel of a patient until the distal end is located at the site of interest to be monitored. With suitable power supplied, the pressure at the site of interest is then monitored by the detector circuitry and displayed or recorded, as with meter 60. Since no air reference lumen is located within the catheter 50, absolute pressure is measured. This, then, permits a catheter structure to be of a very small diameter, such as an inner diameter of less than 1 mm. As discussed previously, the width of the substrate block 10 on which the blister structure is mounted may be on the order of 600 micrometers. Thus, the structure may be installed in a catheter tip of such small dimensions.

Reference is now made to Fig. 9, which illustrates the catheter tip pressure transducer in greater detail. As seen, the housing 54 may take the form of a tubular structure which is preferably rigid relative to the catheter 50 with which it is secured at one end, as with a suitable epoxy or the like. The housing 54 is provided with a suitable side port pressure inlet aperture 56 which extends through its side wall providing communication of pressure from a site of interest to the interior of housing 54. Within the housing there is provided a subassembly including a substrate block 10, made of silicon, which may be secured in place, as with a suitable silicon rubber adhesive 58. The substrate 10 carries the blister structure 9 and is located so that it is exposed to the aperture 56 whereby pressure variations in the blood vessel or the like may be communicated to the flexible roof 14 of the blister FET structure. Suitable connecting wires 61, 62 and 64 are electrically connected to contact pads 30, 34 and 38 (see Fig. 4) and then extend through the lumen of the catheter 50 to the power supply and detector circuit 58.

In summation, by constructing a pressure responsive element in accordance with the present invention, the cross sectional width dimensions may be considerably reduced from that of the prior art, while achieving sufficient pressure responsiveness so that the pressure sensor may be employed in catheters and needles having reduced inner diameters, such as less than 1 mm. It is to be appreciated that various modifications may be made without departing from the spirit and scope of the invention as defined by the appended claims.

CLAIMS

1. A capacitive pressure sensor comprising:

a field effect transistor including a semiconductor substrate having a source region and a drain region and a channel region between said source and drain regions;

a blister structure mounted on said substrate, said blister structure having walls defining a hermetically sealed cavity exhibiting a low pressure less than that of atmospheric, said cavity overlies at least portions of said source and drain regions and the channel region therebetween; and

said cavity walls including a flexible roof containing a layer of conductive material serving as a movable capacitor plate which is spaced from and overlies said channel region so as to vary the transconductance thereof as a function of any displacement of said flexible roof relative to said channel region caused by pressure applied to said roof.

2. A capacitive pressure sensor as set forth in claim 1, wherein said cavity walls include upstanding side walls and a bottom wall which together with said roof encompass said cavity and define an area overlying said source and drain regions.

3. A capacitive pressure sensor as set forth in claim 2, wherein said substrate has a first conductive region laterally spaced from said cavity defining side walls and being electrically isolated from said source and drain regions, said first conductive region is electrically connected to said movable capacitor plate and serves as an access for a gate electrode.

4. A capacitive pressure sensor as set forth in claim 2, wherein said bottom wall is a layer of insulating material, and said side walls and said roof are defined by a lamination including inner and outer layers of insulating material with a layer of conductive material therebetween inclusive of said capacitor plate in said roof.

5. A capacitive pressure sensor as set forth in claim 4 including blister side walls supporting means in said substrate for supporting saidblister side walls while electrically isolating said conductive material from said source and drain regions.

6. A capacitive pressure sensor as set forth in claim 5, wherein said substrate is of a first conductivity type and said source and drain regions are of a second conductivity type, said blister side wall supporting means including support regions of said second conductivity type in said substrate and spaced from said source and drain regions.

7. A capacitive pressure sensor as set forth in claim 6, including channel stopper means interposed between said support regions and said drain and source regions.

8. A capacitive pressure sensor as set forth in claim 7, wherein said substrate is silicon and said layer of conductive material is polysilicon and said layers of insulating material are silicon dioxide and said cavity is hermetically sealed through annealing in oxygen at a temperature on the order of 1000°C to 1150°C.

9. A capacitive pressure sensor as set forth in claim 1, including a needle-like tubular housing having a side port aperture therein, means for mounting said substrate within said housing so that said flexible roof of said blister structure faces said aperture so as to be exposed to pressure acting therethrough.

10. A capacitive pressure sensor as set forth in claim 9, wherein said tubular housing is a catheter tip transducer housing adapted to be fitted to a catheter and being of a size sufficiently small to be inserted into a blood vessel of a living body for measuring blood pressure at a site therein.

11. A method of constructing a capacitive pressure sensor comprising the steps of: providing a semiconductor substrate of a first conductivity type;

forming spaced apart drain and source regions of a second conductivity type in said substrate so as to define a channel region therebetween;

placing a spacer of silicon dioxide on said substrate so that it overlies said channel region and at least portions of said drain and source regions;

applying a layer of polysilicon over said spacer and at least portions of said substrate;

etching away said spacer block so as to leave a cavity over said channel region;

subjecting the thus defined structure to oxidation at a temperature sufficient for oxygen in the cavity to be consumed to a low pressure, less than atmospheric, resulting in a hermetically sealed cavity.

12. A method as set forth in claim 9, wherein said oxidation step takes place at a temperature sufficient to prevent any water to remain in said cavity.

13. A method as set forth in claim 10, wherein said oxidation step takes place at a temperature on the order of 1000°C to 1150 °C.

FIG.1A
FIG.1B
FIG.1C
FIG.1D
FIG.1E
FIG.2A
FIG.2B
G
D
S
P

FIG.3
ATM. PR. +
300 mm Hg.
ATMOSPHERIC
PRESSURE
$I_{ds}$
0
$V_{GS}$

FIG.4
10
28
9
7
5
5
6
6
7
7
32
26
29
36
34
30
38

FIG.5
14
3
8
12
3
12
22
n+
p+
p+
p+
p+
p+
p+
6
23
6
20
7
24
7
n−Si

FIG.6
14
3
22
n+
8
p+
p+
6
20
7
n−Si
10
FIG.7
3
n+
22
p+
p+
p+
6
22
26
22
7
n−Si
10
W

FIG.8
50
54
56
52
58
60
FIG.9
54
56
9
67 64
58
10
62

European Patent Office

# EUROPEAN SEARCH REPORT

0 230 084

EP 86 20 2377

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | IEEE TRANSACTIONS ON BIOMEDICAL ENGINEERING, vol. BME-20, July 1973, pages 312-314, New York, US; W.D. FROBENIUS et al.: "Microminiature solid-state capacitive blood pressure transducer with improved sensitivity" * Whole document * | 1,11 | G 01 L 9/12 |
| D,A | FR-A-1 568 487 (CORNING GLASS WORKS) * Page 5, lines 9-37; page 6; figures * | 1,11 | |
| D,A | FR-A-1 461 244 (INTERNATIONAL STANDARD ELECTRIC CORPORATION) * Whole document * | 1,11 | **TECHNICAL FIELDS SEARCHED (Int. Cl.4)** |
| D,A | EP-A-0 087 264 (THE GENERAL ELECTRIC COMPANY) * Page 5, lines 11-35; page 7, lines 2-14; figures * | 1,11 | G 01 L 9<br>G 01 L 1 |
| D,A | US-A-4 495 820 (S.SHIMADA et al.) * Whole document * | 1,11 | |

The present search report has been drawn up for all claims

| Place of search | Date of completion of the search | Examiner |
|---|---|---|
| THE HAGUE | 24-03-1987 | VAN ASSCHE P.O. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document